# EUROPEAN PATENT SPECIFICATION

(11) **EP 3 584 438 B1**
(45) Date of publication and mention of the grant of the patent: **11.09.2024**
(21) Application number: 19181306.2
(22) Date of filing: 19.06.2019
(51) Int. Cl.: F03D 17/00, F03D 80/50, F03D 80/70

(54) **SYSTEM AND METHOD FOR DETERMINING WATER CONTAMINATION IN OIL OF A WIND TURBINE GEARBOX**
SYSTEM UND VERFAHREN ZUR BESTIMMUNG DER WASSERVERSCHMUTZUNG IN ÖL EINES WINDTURBINENGETRIEBES
SYSTÈME ET PROCÉDÉ PERMETTANT DE DÉTERMINER LA CONTAMINATION DE L'EAU DANS L'HUILE D'UNE BOÎTE DE VITESSES D'ÉOLIENNE

(30) Priority: 20.06.2018 US 201816012925
(43) Date of publication of application: 25.12.2019
(73) Proprietor: General Electric Renovables España, S.L., 08005 Barcelona (ES)
(72) Inventor: Garry, Michael, Greenville, SC South Carolina 29615 (US); McLeod, Robert A., Greenville, SC South Carolina 29615 (US); Cencula, James Edward, Greenville, SC South Carolina 29615 (US); Jaiswal, Yash Dayashankar, 560066 Bangalore (IN); Adhiachari, Subramani, 560066 Bangalore (IN)
(74) Representative: Zimmermann & Partner Patentanwälte mbB

(56) References cited:
- ES-A1- 2 361 100
- JP-A- 2012 180 921
- US-A1- 2013 129 512
- US-A1- 2017 138 922

## Description

### FIELD

The present disclosure relates in general to wind turbines, and more particularly to a system and method for determining water contamination in oil of a wind turbine gearbox.

### BACKGROUND

Generally, a wind turbine includes a tower, a nacelle mounted on the tower, and a rotor coupled to the nacelle. The rotor generally includes a rotatable hub and a plurality of rotor blades coupled to and extending outwardly from the hub. Each rotor blade may be spaced about the hub so as to facilitate rotating the rotor to enable kinetic energy to be converted into usable mechanical energy, which may then be transmitted to an electric generator disposed within the nacelle for the production of electrical energy. Typically, a gearbox is used to drive the electric generator in response to rotation of the rotor. For instance, the gearbox may be configured to convert a low speed, high torque input provided by the rotor to a high speed, low torque output that may drive the electric generator.

The gearbox generally includes a gearbox housing containing a plurality of gears (e.g., planetary, ring and/or sun gears as well as non-planetary gears) connected via one or more planetary carriers and bearings for converting the low speed, high torque input of the rotor shaft to a high speed, low torque output for the generator. In addition, each of the gears rotates about a pin shaft arranged within the one or more planetary carriers. Further, a journal bearing is generally positioned around each of the pin shafts between a respective pin shaft and a rotating gear. In addition, lubrication via oil is generally provided between the various bearings and/or the rotating gears.

During operation, in onshore and offshore wind turbines alike, the oil in the gearbox can become contamination with water. Such water compromises the integrity of the oil, thereby decreasing component life. As such, oil sampling is generally required at certain intervals over the lifetime of the wind turbine to monitor the health of the oil as well as the gearbox. Typical oil sampling requires collection from a remote site and shipping of the collection to a laboratory for testing, which can be expensive. In addition, even though the amount requirement for replacement is minimal, the overall replacement process can be time-consuming and/or costly depending on the location of the wind turbine. However, the consequence of continuing to operate the gearbox with contaminated oil is even more costly. Reference US 2017/0138922 A1 describes, for example, a system for a wind turbine which includes a resonant sensor being arranged in contact with oil within a gearbox for calculating a degradation value for the gearbox.

Accordingly, the present disclosure is directed to a system and method for predicting water contamination in oil of a wind turbine gearbox so as to address the aforementioned issues.

### BRIEF DESCRIPTION

Aspects and advantages of the invention will be set forth in part in the following description, or may be obvious from the description, or may be learned through practice of the invention

In one aspect, the present disclosure is directed to a method for determining an amount of water contamination in oil of a gearbox of a wind turbine. It should be understood that the method may be applied to both off-shore and on-shore wind turbines. The method includes receiving, via a controller, one or more weather conditions at the wind turbine. The method also include receiving, via the controller, one or more operational parameters of the wind turbine. Further, the method includes calculating, via the controller, the amount of water contamination in the oil of the gearbox as a function of the one or more weather conditions at the wind turbine and of the one or more operational parameters of the wind turbine In addition, the method includes implementing a corrective action based on the calculated amount of water contamination in the oil of the gearbox.

In one embodiment, the step of calculating the amount of water contamination in the oil of the gearbox as a function of the one or more operational parameters of the wind turbine and the one or more weather conditions at the wind turbine may include inputting the one or more operational parameters and the one or more weather conditions into at least one physics-based model. For example, in such embodiments, the physics-based model(s) may include an adsorption isotherm model for desiccant, an evaporation model for water from oil, a water vapor pressure model, a water solubility model in oil, a relative humidity to water vapor fraction model, a water mass balance model for air and oil, a leakage flow model for one or more labyrinth seals, an air flow model for air breather, or any other suitable model.

According to the embodiment, the operational parameter(s) includes generator speed, a temperature within a nacelle of the wind turbine, a temperature of the oil, differential pressure across the gearbox, oil type, gearbox volume, desiccant specifications, labyrinth leakage flow, water balance in the oil, an evaporation rate from the water in the oil, absorption, adsorption in desiccant, condensation of the water, and/or water dissolution, or similar or combinations thereof.

According to the embodiment, the weather condition(s) at the wind turbine include air pressure, air temperature, humidity, dew point, wind speed, wind direction, wind turbulence, or any other weather parameter or combinations thereof.

In additional embodiments, the step of implementing the corrective action based on the calculated amount of water contamination in the oil of the gearbox may include scheduling a maintenance procedure. In such embodiments, the step of scheduling the maintenance procedure may include notifying a user, triggering an automated notification or alarm, scheduling an oil sampling procedure, scheduling an oil filtration procedure, replacing a desiccant of the gearbox, replacing the oil in the gearbox, replacing the gearbox, and/or any other suitable maintenance procedures.

In several embodiments, the method may include calculating a remaining desiccant life, a remaining oil life, and/or a remaining bearing life based on the amount of water contamination in the oil of the gearbox.

In another aspect, the present disclosure is directed to a system for determining an amount of water contamination in oil of a gearbox of a wind turbine. The system includes at least one sensor for monitoring one or more operational parameters of the wind turbine and one or more weather conditions at the wind turbine. In addition, the system includes at least one controller communicatively coupled to the sensor(s). As such, the controller includes one or more processors and one or more memory devices. Thus, the memory device(s) are configured to store computer-readable instructions that when executed by the processor(s) cause the processor(s) to perform one or more operations, including but not limited to receiving the one or more operational parameters of the wind turbine, receiving the one or more weather conditions at the wind turbine, calculating an amount of water contamination in the oil of the gearbox as a function of the one or more operational parameters of the wind turbine and the one or more weather conditions at the wind turbine, and implementing a corrective action based on the calculated amount of water contamination in the oil of the gearbox. It should also be understood that the system may further include any of the additional features described herein.

In yet another aspect, the present disclosure is directed to a method for determining an amount of water contamination in oil of a gearbox of a wind turbine. The method includes receiving, via a controller, one or more weather conditions at the wind turbine. The weather condition(s) include air pressure, air temperature, humidity, dew point, wind speed, wind direction, and/or wind turbulence. The method also includes calculating, via the controller, the amount of water contamination in the oil of the gearbox as a function of the one or more weather conditions at the wind turbine. Further, the method includes implementing a corrective action based on the calculated amount of water contamination in the oil of the gearbox. It should also be understood that the method may further include any of the additional features and/or steps described herein.

These and other features, aspects and advantages of the present invention will become better understood with reference to the following description and appended claims. The accompanying drawings, which are incorporated in and constitute a part of this specification, illustrate embodiments of the invention and, together with the description, serve to explain the principles of the invention.

### BRIEF DESCRIPTION OF THE DRAWINGS

A full and enabling disclosure of the present invention, including the best mode thereof, directed to one of ordinary skill in the art, is set forth in the specification, which makes reference to the appended figures, in which:
FIG. 1 illustrates a perspective view of one embodiment of a wind turbine according to the present disclosure;
FIG. 2 illustrates a detailed, internal view of one embodiment of a nacelle of a wind turbine according to the present disclosure;
FIG. 3 illustrates a block diagram of one embodiment of a controller of a wind turbine according to the present disclosure;
FIG. 4 illustrates a cross-sectional view of one embodiment of a gearbox assembly of a wind turbine according to the present disclosure; and
FIG. 5 illustrates a flow diagram of one embodiment of a method for determining water contamination in oil of a gearbox of a wind turbine according to the present disclosure.

### DETAILED DESCRIPTION

Reference now will be made in detail to embodiments of the invention, one or more examples of which are illustrated in the drawings. Each example is provided by way of explanation of the invention, not limitation of the invention. For instance, features illustrated or described as part of one embodiment can be used with another embodiment to yield a still further embodiment. Thus, it is intended that the present invention covers such modifications and variations as come within the scope of the appended claims.

Generally, the present disclosure is directed to a system and method for predicting water in oil contamination for onshore and/or off-shore wind turbines alike. In one embodiment, the system uses a combination of sensor data from the wind turbine, including but not limited to air pressure, air temperature, wind speed, generator speed, nacelle temperature, gearbox oil temperature, desiccant specifications, and/or local weather information as inputs to one or more physics-based models for predicting the rate of contamination of water in the gearbox oil. In addition, additional parameters, such as labyrinth leakage flow, water balance in the oil and/or the atmosphere, evaporation of water from the water in the oil emulsion, absorption, adsorption in desiccant, condensation of the water, and/or water dissolution, may also be included in the physics-based model(s). Thus, the system and method of the present disclosure uses local weather data (e.g. primarily relative humidity) to predict the water contamination in the gearbox oil. Further, the system and method of the present disclosure can eliminate and/or optimize the conventional oil sampling and also improves condition-based maintenance (i.e. replacing the oil only when needed).

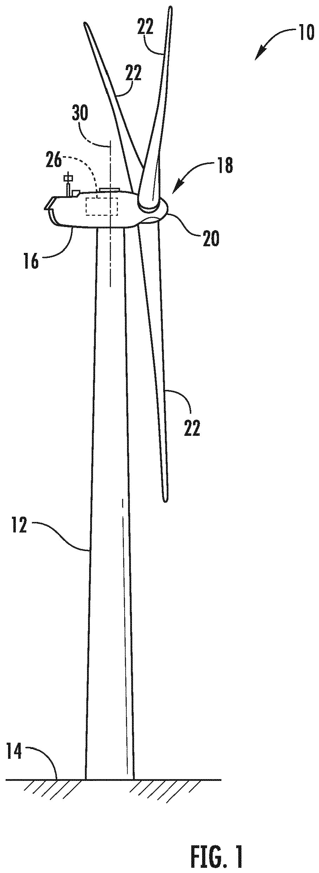

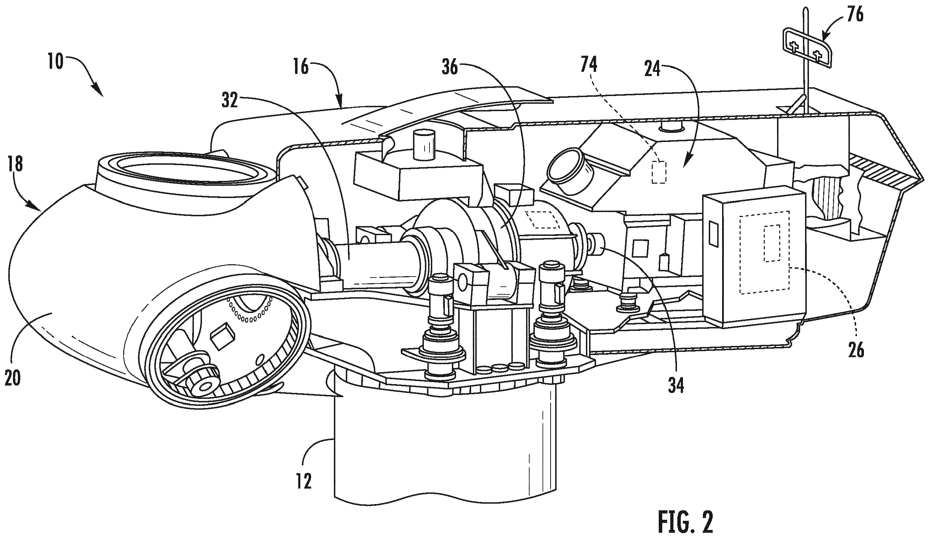

Referring now to the drawings, FIG. 1 illustrates a perspective view of one embodiment of a wind turbine 10 according to the present disclosure. As shown, the wind turbine 10 includes a tower 12 extending from a support surface 14, a nacelle 16 mounted on the tower 12, and a rotor 18 coupled to the nacelle 16. It should be understood that the wind turbine 10 described herein may include both off-shore and on-shore wind turbines. The rotor 18 includes a rotatable hub 20 and at least one rotor blade 22 coupled to and extending outwardly from the hub 20. For example, in the illustrated embodiment, the rotor 18 includes three rotor blades 22. However, in an alternative embodiment, the rotor 18 may include more or less than three rotor blades 22. Each rotor blade 22 may be spaced about the hub 20 to facilitate rotating the rotor 18 to enable kinetic energy to be transferred from the wind into usable mechanical energy, and subsequently, electrical energy. For instance, the hub 20 may be rotatably coupled to an electric generator 24 (FIG. 2) positioned within the nacelle 16 to permit electrical energy to be produced.

As shown, the wind turbine 10 may also include a turbine control system or a turbine controller 26 centralized within the nacelle 16. For example, as shown in FIG. 2, the turbine controller 26 is disposed within a control cabinet mounted to a portion of the nacelle 16. However, it should be appreciated that the turbine controller 26 may be disposed at any location on or in the wind turbine 10, at any location on the support surface 14 or generally at any other location. In general, the turbine controller 26 may be configured to transmit and execute wind turbine control signals and/or commands in order to control the various operating modes (e.g., start-up or shut-down sequences) and/or components of the wind turbine 10.

Referring now to FIG. 2, a simplified, internal view of a nacelle 16 of the wind turbine 10 according to conventional construction is illustrated. As shown, the generator 24 may be disposed within the nacelle 16. In general, the generator 24 may be coupled to the rotor 18 of the wind turbine 10 for producing electrical power from the rotational energy generated by the rotor 18. For example, as shown in the illustrated embodiment, the rotor 18 may include a rotor shaft 32 coupled to the hub 20 for rotation therewith. The rotor shaft 32 may, in turn, be rotatably coupled to a generator shaft 34 of the generator 24 through a gearbox assembly 36. As is generally understood, the rotor shaft 32 may provide a low speed, high torque input to the gearbox assembly 36 in response to rotation of the rotor blades 22 and the hub 20. The gearbox assembly 36 may then be configured to convert the low speed, high torque input to a high speed, low torque output to drive the generator shaft 34 and, thus, the generator 24. In alternative embodiments, the rotor shaft 32 may be eliminated and the rotatable hub 20 may be configured to turn the gears of the gearbox assembly 36, rather than requiring a separate rotor shaft 32.

In addition, as shown in FIG. 2, one or more sensors 74, 76 may be provided on the wind turbine 10. More specifically, as shown, a sensor 74 may be provided at any suitable location on or within the wind turbine 10 to measure various operational parameters thereof. For example, as shown, the sensor 74 is associated with the generator so as to measure generator speed. Still further sensors may be provided to monitor additional operational parameter(s) including but not limited to a temperature within the nacelle 16, a temperature of the oil, differential pressure across the gearbox, oil type, gearbox volume, desiccant specifications, labyrinth leakage flow, water balance in the oil and/or an atmosphere surrounding the wind turbine 10, an evaporation rate from the water in the oil, absorption, adsorption in desiccant, condensation of the water, and/or water dissolution, or similar or combinations thereof.

In addition, as shown, a wind sensor 76 may be provided on the wind turbine 10. The wind sensor 76, which may for example be a wind vane, and anemometer, and LIDAR sensor, or another suitable sensor, may measure various weather conditions including but not limited to air pressure, air temperature, humidity, dew point, wind speed, wind direction, wind turbulence, or any other weather parameter or combinations thereof.

The sensors 74, 76 described herein may further be in communication with the controller 26, and may provide related information to the controller 26. It should also be appreciated that, as used herein, the term "monitor" and variations thereof indicates that the various sensors of the wind turbine 10 may be configured to provide a direct measurement of the parameters being monitored or an indirect measurement of such parameters. Thus, the sensors described herein may, for example, be used to generate signals relating to the parameter being monitored, which can then be utilized by the controller 26 to determine the condition.

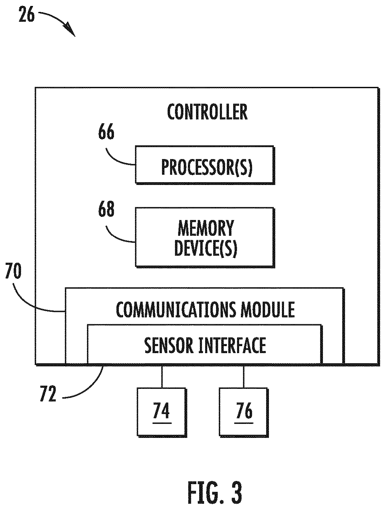

Referring now to FIG. 3, the turbine controller 26 may include one or more processor(s) 66 and associated memory device(s) 68 configured to perform a variety of computer-implemented functions (e.g., performing the methods, steps, calculations and the like and storing relevant data as disclosed herein) for controlling the wind turbine 10. Additionally, the controller 26 may also include a communications module 70 to facilitate communications between the module 70 and the various components of the wind turbine. Further, the communications module 70 may include a sensor interface 72 (e.g., one or more analog-to-digital converters) to permit signals transmitted from one or more sensors 74, 76 to be converted into signals that can be understood and processed by the processor(s) 66. It should be appreciated that the sensors 74, 76 may be communicatively coupled to the communications module 70 using any suitable means. For example, as shown in FIG. 3, the sensors 74, 76 may be coupled to the sensor interface 72 via a wired connection. However, in other embodiments, the sensors 74, 76 may be coupled to the sensor interface 72 via a wireless connection, such as by using any suitable wireless communications protocol known in the art. As such, the processor 66 may be configured to receive one or more signals from the sensors 74, 76.

As used herein, the term "processor" refers not only to integrated circuits referred to in the art as being included in a computer, but also refers to a controller, a microcontroller, a microcomputer, a programmable logic controller (PLC), an application specific integrated circuit, and other programmable circuits. The processor 66 is also configured to compute advanced control algorithms and communicate to a variety of Ethernet or serial-based protocols (Modbus, OPC, CAN, etc.). Additionally, the memory device(s) 68 may generally comprise memory element(s) including, but not limited to, computer readable medium (e.g., random access memory (RAM)), computer readable non-volatile medium (e.g., a flash memory), a floppy disk, a compact disc-read only memory (CD-ROM), a magnetooptical disk (MOD), a digital versatile disc (DVD) and/or other suitable memory elements. Such memory device(s) 68 may generally be configured to store suitable computer-readable instructions that, when implemented by the processor(s) 66, configure the controller 26 to perform the various functions as described herein.

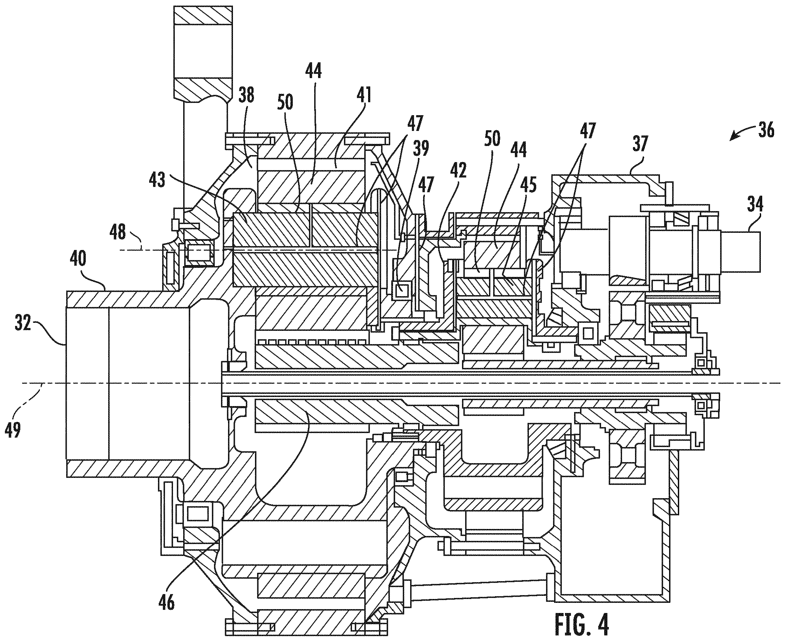

Referring now to FIG. 4, a cross-sectional view of one embodiment of the gearbox assembly 36 according to the present disclosure is illustrated. As shown, the gearbox assembly 36 includes a gear assembly 38, such as a gear assembly, housed within a gearbox housing 37. More specifically, the gear assembly 38 includes a plurality of gears (e.g., planetary, ring, sun, helical, and/or spur gears) and bearings 39 for converting the low speed, high torque input of the rotor shaft 32 to a high speed, low torque output for the generator 24. For example, as shown, the input shaft 32 may provide an input load to the gear assembly 38 and the system 38 may provide an output load to the generator 24 (FIG. 2) as is generally known in the art. Thus, during operation, input load at an input rotational speed is transmitted through the gear assembly 38 and provided as output load at output rotational speed to the generator 24.

Further, the gear assembly 38 includes a first planetary carrier 40 and a second planetary carrier 42 operatively coupling a plurality of gears. Further, as shown, the gear assembly 38 includes, at least, a ring gear 41, one or more planet gears 44, a sun gear 46, one or more first pin shafts 43, and one or more second pin shafts 45. For example, in several embodiments, the gear assembly 38 may include one, two, three, four, five, six, seven, eight, or more planet gears 44. Further, each of the gears 41, 44, 46 includes a plurality of teeth. The teeth are sized and shaped to mesh together such that the various gears 41, 44, 46 engage each other. For example, the ring gear 41 and the sun gear 46 may each engage the planet gears 44. In addition, it should be understood that the gears 41, 44, 46 described herein may include any suitable type of gears, including but not limited to spur gears, face gears, helical gears, double helical gears, or similar.

In some embodiments, one or both of the planetary carriers 40, 42 may be stationary. In these embodiments, the input shaft 32 may be coupled to the ring gear 41, and input loads on the input shaft 32 may be transmitted through the ring gear 41 to the planet gears 44. Thus, the ring gear 41 may drive the gear assembly 38. In other embodiments, the ring gear 41 may be stationary. In these embodiments, the input shaft 32 may be coupled to the planetary carriers 40, 42, and input loads on the input shaft 32 may be transmitted through the planetary carriers 40, 42 to the planet gears 44. Thus, the planetary carriers 40, 42 may drive the gear assembly 38. In still further embodiments, any other suitable component, such as the planet gear 44 or the sun gear 46, may drive the gear assembly 38.

Still referring to FIG. 4, the sun gear 46 defines a central axis 49, and thus rotates about this central axis 49. The ring gear 41 may at least partially surround the sun gear 46, and be positioned along the central axis 49. Further, the ring gear 41 may (if rotatable) thus rotate about the central axis 49. Each of the planet gears 44 may be disposed between the sun gear 46 and the ring gear 41, and may engage both the sun gear 46 and the ring gear 41. For example, the teeth of the gears may mesh together, as discussed above. Further, each of the planet gears 44 may define a central planet axis 48, as shown. Thus, each planet gear 44 may rotate about its central planet axis 48. Additionally, the planet gears 44 and central planet axes 48 thereof may rotate about the central axis 49.

The gearbox assembly 36 may also include a lubrication system or other means for circulating oil throughout the gearbox components. For example, as shown in FIG. 4, the gearbox assembly 36 may include a plurality of oil passages 47 that are configured to transfer oil therethrough. As is generally understood, the oil may be used to reduce friction between the moving components of the gearbox assembly 36 and may also be utilized to provide cooling for such components, thereby decreasing component wear and other losses within the gearbox assembly 36 and increasing the lifespan thereof. In addition, the oil may contain properties that prevent corrosion of the internal gearbox components.

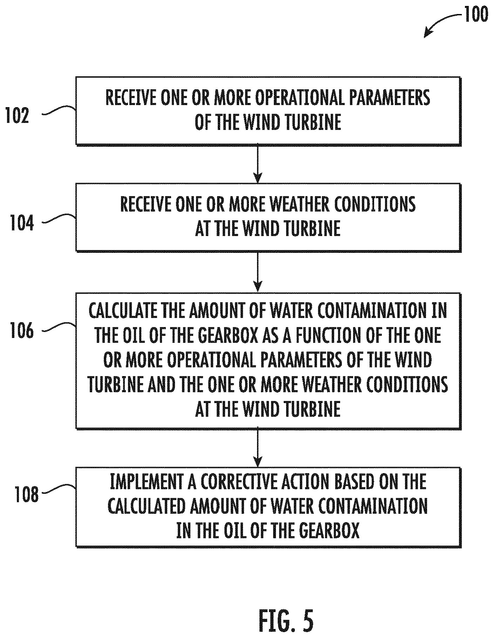

Referring now to FIG. 5, a flow diagram of a specific embodiment of a control algorithm 100 that may be executed for determining an amount of water contamination in oil of a gearbox of a wind turbine is illustrated in accordance with aspects of the present subject matter. In general, the control algorithm 100 will be described herein with reference to the gearbox assembly 36 shown in FIGS 2 and 4. However, in other embodiments, the algorithm 100 may be used in connection with any other suitable implement having any other suitable implement configuration and/or with any other suitable system having any other suitable system configuration.

It should be appreciated that, although FIG. 5 depicts control steps or functions performed in a particular order for purposes of illustration and discussion, the control algorithms discussed herein are not limited to any particular order or arrangement. One skilled in the art, using the disclosures provided herein, will appreciate that various steps or functions of the algorithms disclosed herein can be omitted, rearranged, combined, and/or adapted in various ways without deviating from the scope of the present disclosure.

As shown at 102, the method 100 includes receiving one or more operational parameters of the wind turbine 10, e.g. via the controller 26. For example, as mentioned, the operational parameter(s) may include generator speed, a temperature within the nacelle 16, a temperature of the oil, differential pressure across the gearbox, oil type, gearbox volume, desiccant specifications, labyrinth leakage flow, water balance in the oil and/or an atmosphere surrounding the wind turbine, an evaporation rate from the water in the oil, absorption, adsorption in desiccant, condensation of the water, and/or water dissolution, or similar or combinations thereof.

As shown at 104, the method 100 also includes receiving one or more weather conditions at the wind turbine 10, e.g. the controller 26. More specifically, in certain embodiments, the weather condition(s) at the wind turbine 10 may include air pressure, air temperature, humidity, dew point, wind speed, wind direction, wind turbulence, or any other weather parameter or combinations thereof.

Thus, as shown at 106, the controller 26 is configured to calculate the amount of water contamination in the oil of the gearbox 36 as a function of the operational parameter(s) of the wind turbine 10 and the weather condition(s) at the wind turbine 10. In such embodiments, the controller 26 may be configured to receive the operational parameter(s) and the weather condition(s) as inputs for at least one physics-based model. As used herein, a physics-based model broadly refers to any model used to generate and visualize constrained shapes, motions of rigid and/or non-rigid objects, and/or object interactions with an environment. As such, in particular embodiments, the physics-based model(s) of the present disclosure may include an adsorption isotherm model for a desiccant, an evaporation model for the water from the oil, a water vapor pressure model, a water solubility model in the oil, a relative humidity to water vapor fraction model, a water mass balance model for the air and the oil, a leakage flow model for one or more labyrinth seals of the gearbox 36, an air flow model for air breather, and/or any other suitable model that correlates weather data to the gearbox oil.

Accordingly, still referring to FIG. 5, as shown at 108, the controller 26 is configured to implement a corrective action based on the calculated amount of water contamination in the oil of the gearbox 36. More specifically, in certain embodiments, the controller 26 may be configured to schedule a maintenance procedure. For example, in such embodiments, the controller 26 may be configured to notify a user, trigger an automated notification or alarm, schedule an oil sampling procedure, schedule an oil filtration procedure, replace a desiccant of the gearbox, replace the oil in the gearbox, replace the gearbox, and/or schedule any other suitable maintenance procedures. In addition, the method 100 may also include calculating a remaining desiccant life, a remaining oil life, and/or a remaining bearing life based on the amount of water contamination in the oil of the gearbox 36.

Further described are embodiments which do not show all features of the claimed invention .

A method for determining an amount of water contamination in oil of a gearbox of a wind turbine, the method comprising:
receiving, via a controller, one or more operational parameters of the wind turbine;
receiving, via the controller, one or more weather conditions at the wind turbine;
calculating, via the controller, the amount of water contamination in the oil of the gearbox as a function of the one or more operational parameters of the wind turbine and the one or more weather conditions at the wind turbine; and,
implementing, via the controller, a corrective action based on the calculated amount of water contamination in the oil of the gearbox.

An exemplary embodiment according to the method, wherein calculating the amount of water contamination in the oil of the gearbox as a function of the one or more operational parameters of the wind turbine and the one or more weather conditions at the wind turbine further comprises inputting the one or more operational parameters and the one or more weather conditions into at least one physics-based model.

An exemplary embodiment according to the method, wherein the at least one physics-based model comprises at least one of an adsorption isotherm model for desiccant, an evaporation model for water from oil, a water vapor pressure model, a water solubility model in oil, a relative humidity to water vapor fraction model, a water mass balance model for air and oil, a leakage flow model for one or more labyrinth seals, or an air flow model for air breather.

An exemplary embodiment according to the method, wherein the one or more operational parameters comprise at least one of generator speed, a temperature within a nacelle of the wind turbine, a temperature of the oil, differential pressure across the gearbox, oil type, gearbox volume, desiccant specifications, labyrinth leakage flow, water balance in the oil and/or an atmosphere surrounding the wind turbine, an evaporation rate from the water in the oil, absorption, adsorption in desiccant, condensation of the water, and/or water dissolution.

An exemplary embodiment according to the method, wherein the one or more weather conditions at the wind turbine comprise at least one of air pressure, air temperature, humidity, dew point, wind speed, wind direction, or wind turbulence.

An exemplary embodiment according to the method, wherein implementing the corrective action based on the calculated amount of water contamination in the oil of the gearbox further comprises scheduling a maintenance procedure.

An exemplary embodiment according to the method, wherein scheduling the maintenance procedure further comprises at least one of notifying a user, triggering an automated notification or alarm, scheduling an oil sampling procedure, scheduling an oil filtration procedure, replacing a desiccant of the gearbox, replacing the oil in the gearbox, or replacing the gearbox.

An exemplary embodiment according to the method, further comprising calculating, via the controller, at least one of a remaining desiccant life, a remaining oil life, or a remaining bearing life based on the amount of water contamination in the oil of the gearbox.

An exemplary embodiment according to the method, wherein the wind turbine comprises an off-shore wind turbine.

A system for determining an amount of water contamination in oil of a gearbox of a wind turbine, the system comprising:
at least one sensor for monitoring one or more operational parameters of the wind turbine and one or more weather conditions at the wind turbine; and,
at least one controller communicatively coupled to the at least one sensor, the at least one controller comprising one or more processors and one or more memory devices,
the one or more memory devices configured to store computer-readable instructions that when executed by the one or more processors cause the one or more processors to perform one or more operations, the one or more operations comprising:
   receiving the one or more operational parameters of the wind turbine;
   receiving the one or more weather conditions at the wind turbine;
   calculating an amount of water contamination in the oil of the gearbox as a function of the one or more operational parameters of the wind turbine and the one or more weather conditions at the wind turbine; and,
   implementing a corrective action based on the calculated amount of water contamination in the oil of the gearbox.

An exemplary embodiment of the system, wherein calculating the amount of water contamination in the oil of the gearbox as a function of the one or more operational parameters of the wind turbine and the one or more weather conditions at the wind turbine further comprises inputting the one or more operational parameters and the one or more weather conditions into at least one physics-based model.

An exemplary embodiment of the system, wherein the at least one physics-based model comprises at least one of an adsorption isotherm model for desiccant, an evaporation model for water from oil, a water vapor pressure model, a water solubility model in oil, a relative humidity to water vapor fraction model, a water mass balance model for air and oil, a leakage flow model for one or more labyrinth seals, or an air flow model for air breather.

An exemplary embodiment of the system, wherein the one or more operational parameters comprise at least one of generator speed, a temperature within a nacelle of the wind turbine, a temperature of the oil, differential pressure across the gearbox, oil type, gearbox volume, desiccant specifications, labyrinth leakage flow, water balance in the oil and/or an atmosphere surrounding the wind turbine, an evaporation rate from the water in the oil, absorption, adsorption in desiccant, condensation of the water, and/or water dissolution.

An exemplary embodiment of the system, wherein the one or more weather conditions at the wind turbine comprise at least one of air pressure, air temperature, humidity, dew point, wind speed, wind direction, wind turbulence.

An exemplary embodiment of the system, wherein implementing the corrective action based on the calculated amount of water contamination in the oil of the gearbox further comprises scheduling a maintenance procedure.

An exemplary embodiment of the system, wherein the maintenance procedure comprises at least one of notifying a user, triggering an automated notification or alarm, scheduling an oil sampling procedure, scheduling an oil filtration procedure, replacing a desiccant of the gearbox, replacing the oil in the gearbox, or replacing the gearbox.

An exemplary embodiment of the system, wherein the one or more operations further comprise calculating, via the controller, at least one of a remaining desiccant life, a remaining oil life, or a remaining bearing life based on the amount of water contamination in the oil of the gearbox.

An exemplary embodiment of the system, wherein the wind turbine comprises an off-shore wind turbine.

A exemplary embodiment of a further method for determining an amount of water contamination in oil of a gearbox of a wind turbine, the method comprising: receiving, via a controller, one or more weather conditions at the wind turbine, the one or more weather conditions comprising at least one of air pressure, air temperature, humidity, dew point, wind speed, wind direction, wind turbulence; calculating, via the controller, the amount of water contamination in the oil of the gearbox as a function of the one or more weather conditions at the wind turbine; and, implementing, via the controller, a corrective action based on the calculated amount of water contamination in the oil of the gearbox.

An exemplary embodiment of the further method, further comprising receiving one or more operational parameters of the wind turbine and calculating the amount of water contamination in the oil of the gearbox as a function of the one or more operational parameters of the wind turbine and the one or more weather conditions at the wind turbine, the one or more operational parameters comprising at least one of generator speed, a temperature within a nacelle of the wind turbine, a temperature of the oil, differential pressure across the gearbox, oil type, gearbox volume, desiccant specifications, labyrinth leakage flow, water balance in the oil and/or an atmosphere surrounding the wind turbine, an evaporation rate from the water in the oil, absorption, adsorption in desiccant, condensation of the water, and/or water dissolution.

This written description uses examples to disclose the invention, including the best mode, and also to enable any person skilled in the art to practice the invention, including making and using any devices or systems and performing any incorporated methods. The patentable scope of the invention is defined by the claims.

## Claims

1. A method for determining an amount of water contamination in oil of a gearbox (36) of a wind turbine (10), the method comprising:
Receiving (104), via a controller (26), one or more weather conditions at the wind turbine (10);
Receiving, via the controller (26), one or more operational parameters of the wind turbine (10);
Calculating (106), via the controller (26), the amount of water contamination in the oil of the gearbox (36) as a function of the one or more operational parameters of the wind turbine (10) and the one or more weather conditions at the wind turbine (10),
and,
Implementing (108), via the controller (26), a corrective action based on the calculated amount of water contamination in the oil of the gearbox (36);
wherein the one or more operational parameters are at least one of generator speed, oil type, gearbox volume, and desiccant specifications, and
wherein the one or more weather conditions at the wind turbine (10) are at least one of humidity, dew point, and wind speed.

2. The method of claim 1, wherein calculating the amount of water contamination in the oil of the gearbox (36) as a function of the one or more operational parameters of the wind turbine (10) and the one or more weather conditions at the wind turbine (10) further comprises inputting the one or more operational parameters and the one or more weather conditions into at least one physics-based model.

3. The method of claim 2, wherein the at least one physics- based model comprises at least one of an adsorption isotherm model for desiccant, an evaporation model for water from oil, a water vapor pressure model, a water solubility model in oil, a relative humidity to water vapor fraction model, a water mass balance model for air and oil, a leakage flow model for one or more labyrinth seals, or an air flow model for air breather.

4. The method of any of the preceding claims, wherein implementing the corrective action based on the calculated amount of water contamination in the oil of the gearbox (36) further comprises scheduling a maintenance procedure.

5. The method of claim 4, wherein scheduling the maintenance procedure further comprises at least one of notifying a user, triggering an automated notification or alarm, scheduling an oil sampling procedure, scheduling an oil filtration procedure, replacing a desiccant of the gearbox (36), replacing the oil in the gearbox (36), or replacing the gearbox (36).

6. The method of any of the preceding claims, further comprising calculating, via the controller (26), at least one of a remaining desiccant life, a remaining oil life, or a remaining bearing life based on the amount of water contamination in the oil of the gearbox (36).

7. The method of any of the preceding claims, wherein the wind turbine (10) comprises an off-shore wind turbine.

8. A system for determining an amount of water contamination in oil of a gearbox (36) of a wind turbine (10), the system comprising:
at least one sensor (74, 76) for monitoring one or more operational parameters of the wind turbine (10) and one or more weather conditions at the wind turbine (10); and,
at least one controller (26) communicatively coupled to the at least one sensor (74, 76), the at least one controller (26) comprising one or more processors (66) and one or more memory devices (68), the one or more memory devices (68) configured to store computer readable instructions that when executed by the one or more processors (66) cause the one or more processors (66) to perform a method according to one of the preceding claims.

## Patentansprüche

1. Verfahren zum Bestimmen einer Wasserverunreinigung im Öl eines Getriebes (36) einer Windturbine (10), wobei das Verfahren umfasst:
Empfangen (104) einer oder mehrerer Wetterbedingungen an der Windturbine (10) über eine Steuerung (26);
Empfangen von einem oder mehreren Betriebsparametern der Windturbine (10) über die Steuerung (26);
Berechnen (106), über die Steuerung (26), der Menge an Wasserverunreinigung im Öl des Getriebes (36) als eine Funktion des einen oder der mehreren Betriebsparameter der Windturbine (10) und der einen oder der mehreren Wetterbedingungen an der Windturbine (10),
und,
Ausführen (108) einer Korrekturmaßnahme, über die Steuerung (26), auf der Grundlage der berechneten Menge an Wasserverunreinigung im Öl des Getriebes (36);
wobei es sich bei dem einen oder den mehreren Betriebsparametern um mindestens einen der folgenden Parameter handelt: Generatordrehzahl, Öltyp, Getriebevolumen und Trockenmittelspezifikationen, und
wobei es sich bei den einen oder mehreren Wetterbedingungen an der Windturbine (10) um mindestens einen der Parameter Luftfeuchtigkeit, Taupunkt und Windgeschwindigkeit handelt.

2. Verfahren nach Anspruch 1, wobei das Berechnen der Menge an Wasserverunreinigung im Öl des Getriebes (36) als Funktion des einen oder der mehreren Betriebsparameter der Windturbine (10) und der einen oder der mehreren Wetterbedingungen an der Windturbine (10) ferner das Eingeben des einen oder der mehreren Betriebsparameter und der einen oder der mehreren Wetterbedingungen in mindestens ein physikalisch basiertes Modell umfasst.

3. Verfahren nach Anspruch 2, wobei das mindestens eines physikalisch basierte Modell mindestens eines umfasst von einem Adsorptionsisothermenmodell für Trockenmittel, einem Verdunstungsmodell für Wasser aus Öl, einem Wasserdampfdruckmodell, einem Modell für die Wasserlöslichkeit in Öl, einem Modell für den Anteil der relativen Luftfeuchtigkeit am Wasserdampf, einem Wassermassenbilanzmodell für Luft und Öl, einem Leckstrommodell für eine oder mehrere Labyrinthdichtungen oder einem Luftstrommodell für einen Entlüfter.

4. Verfahren nach einem der vorhergehenden Ansprüche, wobei die Durchführung der Korrekturmaßnahme auf der Grundlage der berechneten Menge an Wasserverunreinigung im Öl des Getriebes (36) ferner die Planung eines Wartungsvorgangs umfasst.

5. Verfahren nach Anspruch 4, wobei das Planen der Wartungsprozedur außerdem mindestens eines umfasst von: Benachrichtigung eines Benutzers, Auslösen einer automatischen Benachrichtigung oder eines Alarms, Planen einer Ölprobenentnahme, Planen einer Ölfiltrationsprozedur, Ersetzen eines Trockenmittels des Getriebes (36), Ersetzen des Öls im Getriebe (36) oder Ersetzen des Getriebes (36).

6. Verfahren nach einem der vorhergehenden Ansprüche, ferner umfassend die Berechnung einer verbleibenden Lebensdauer des Trockenmittels, einer verbleibenden Lebensdauer des Öls oder einer verbleibenden Lebensdauer des Lagers auf der Grundlage der Menge der Wasserverunreinigung im Öl des Getriebes (36) durch die Steuereinheit (26).

7. Verfahren nach einem der vorhergehenden Ansprüche, wobei die Windturbine (10) eine Off-Shore-Windturbine ist.

8. System zum Bestimmen einer Wasserverunreinigung im Öl eines Getriebes (36) einer Windturbine (10), wobei das System umfasst:
mindestens einen Sensor (74, 76) zur Überwachung eines oder mehrerer Betriebsparameter der Windturbine (10) und einer oder mehrerer Wetterbedingungen an der Windturbine (10); und,
mindestens eine Steuerung (26), die kommunikativ mit dem mindestens einen Sensor (74, 76) gekoppelt ist, wobei die mindestens eine Steuerung (26) einen oder mehrere Prozessoren (66) und eine oder mehrere Speichervorrichtungen (68) umfasst, wobei die eine oder mehreren Speichervorrichtungen (68) so konfiguriert sind, dass sie computerlesbare Anweisungen speichern, die, wenn sie von dem einen oder den mehreren Prozessoren (66) ausgeführt werden, den einen oder die mehreren Prozessoren (66) veranlassen, ein Verfahren nach einem der vorhergehenden Ansprüche durchzuführen.

## Revendications

1. Procédé permettant de déterminer la quantité de contamination par l'eau de l'huile d'un multiplicateur (36) d'une éolienne (10), le procédé comprenant :
la réception (104), par l'intermédiaire d'un automate (26), d'une ou plusieurs conditions météorologiques au niveau de l'éolienne (10) ;
la réception, par l'intermédiaire de l'automate (26), d'un ou plusieurs paramètres opérationnels de l'éolienne (10) ;
le calcul (106), par l'intermédiaire de l'automate (26), de la quantité de contamination par l'eau de l'huile du multiplicateur (36) en fonction d'un ou plusieurs paramètres opérationnels de l'éolienne (10) et d'une ou plusieurs conditions météorologiques au niveau de l'éolienne (10),
et,
la mise en oeuvre (108), par l'intermédiaire de l'automate (26), d'une action corrective basée sur la quantité calculée de contamination par l'eau de l'huile du multiplicateur (36) ;
dans lequel le ou les plusieurs paramètres opérationnels sont au moins l'un parmi la vitesse du générateur, le type d'huile, le volume du multiplicateur et les spécifications du déshydratant, et
dans lequel la ou les plusieurs conditions météorologiques au niveau de l'éolienne (10) sont au moins l'un parmi l'humidité, le point de rosée et la vitesse du vent.

2. Procédé selon la revendication 1, dans lequel le calcul de la quantité de contamination par l'eau de l'huile du multiplicateur (36) en fonction d'un ou plusieurs paramètres opérationnels de l'éolienne (10) et d'une ou plusieurs conditions météorologiques au niveau de l'éolienne (10) comprend en outre l'entrée d'un ou plusieurs paramètres opérationnels et d'une ou plusieurs conditions météorologiques dans au moins un modèle basé sur la physique.

3. Procédé selon la revendication 2, dans lequel au moins un modèle basé sur la physique comprend au moins l'un parmi un modèle d'isotherme d'adsorption pour le déshydratant, un modèle d'évaporation de l'eau de l'huile, un modèle de pression de vapeur d'eau, un modèle de solubilité de l'eau de l'huile, un modèle d'humidité relative à la fraction de vapeur d'eau, un modèle d'équilibre de masse de l'eau pour l'air et l'huile, un modèle de flux de fuite pour un ou plusieurs joints à labyrinthe, ou un modèle de flux d'air pour le reniflard.

4. Procédé selon l'une quelconque des revendications précédentes, dans lequel la mise en oeuvre de l'action corrective basée sur la quantité calculée de contamination par l'eau de l'huile du multiplicateur (36) comprend en outre la programmation d'une procédure de maintenance.

5. Procédé selon la revendication 4, dans lequel la programmation de la procédure de maintenance comprend en outre au moins l'un parmi la notification à un utilisateur, le déclenchement d'une notification ou d'une alarme automatisée, la programmation d'une procédure d'échantillonnage de l'huile, la programmation d'une procédure de filtration de l'huile, le remplacement d'un déshydratant du multiplicateur (36), le remplacement de l'huile dans le multiplicateur (36), ou le remplacement du multiplicateur (36).

6. Procédé selon l'une quelconque des revendications précédentes, comprenant en outre le calcul, par l'intermédiaire de l'automate (26), d'au moins l'une parmi les durées de vie restantes du déshydratant, de l'huile ou des roulements, en fonction de la quantité de contamination par l'eau de l'huile du multiplicateur (36).

7. Procédé selon l'une quelconque des revendications précédentes, dans lequel l'éolienne (10) comprend une éolienne en mer.

8. Système permettant de déterminer la quantité de contamination par l'eau de l'huile d'un multiplicateur (36) d'une éolienne (10), le système comprenant :
au moins un capteur (74, 76) pour surveiller un ou plusieurs paramètres opérationnels de l'éolienne (10) et une ou plusieurs conditions météorologiques au niveau de l'éolienne (10) ; et, au moins un automate (26) couplé de manière communicative à l'au moins un capteur (74, 76), l'au moins un automate (26) comprenant un ou plusieurs processeurs (66) et un ou plusieurs dispositifs de mémoire (68), le ou les plusieurs dispositifs de mémoire (68) étant configurés pour stocker des instructions lisibles par ordinateur qui, lorsqu'elles sont exécutées par le ou les plusieurs processeurs (66), amènent le ou les plusieurs processeurs (66) à exécuter un procédé selon l'une quelconque des revendications précédentes.
